# EUROPEAN PATENT APPLICATION

(11) **EP 1 801 198 A1**
(43) Date of publication of application: **27.06.2007**
(21) Application number: 06251751.1
(22) Date of filing: 30.03.2006
(51) Int. Cl.: C12M 3/00

(54) **Injection apparatus and method**

(30) Priority: 22.12.2005 JP 2005369470
(71) Applicant: Fujitsu Ltd., Kawasaki-shi, Kanagawa 211-8588 (JP)
(72) Inventor: Nishiyama, Shusaku, Fujitsu Limited, Nakahara-ku Kawasaki-shi Kanagawa 211-85 (JP); Nishio, Chikara, Fujitsu Limited, Nakahara-ku Kawasaki-shi Kanagawa 211-85 (JP)
(74) Representative: Stebbing, Timothy Charles

(57) **Abstract**

An injection apparatus that inserts an injection needle (106) into a target region in a minute object (C), and injects a predetermined material into the target region from the injection needle includes a positioning controller (150) that determines, as an insertion direction, a direction that provides the longest length in the target region among plural directions in each of which the injection needle can be inserted into the minute object, and a moving unit (131) that moves the injection needle along the insertion direction.

## Description

The present invention relates generally to an injection apparatus or a microinjection apparatus that injects a material into a minute object, such as a cell and a colloid, via a capillary. The present invention is suitable, for example, for an injection apparatus that captures minute floating cells and injects material into themvia a capillary in a drug discovery system that investigates reactions of biogenetic cells, such as leukocytes' antibody generations, for use with a medical field. The "drug discovery system", as used herein, generally means a system that processes a cell e.g., injects extrinsic gene and medication solutions using a fine needle or a capillary into a cell, then cultivates each processed cell, individually evaluates or processes the cell (e.g., by screening and antibody extraction).

Recently, opportunities of using cells, in which genes or medications are injected, have increased in the field of regenerative medicine and genome-based drug discovery, etc. Unlike in research, it is necessary in medical applications to previously determine a combination between a cell and an introduced material and to independently evaluate each cell, e.g., observe whether or not there is an effect expression in a single cell. For example, medication is injected into each cell and an effect of the medication is evaluated. Since it is necessary to change a dose of medication and a type of injected medication, injections of medications to many cells are demanded. In addition, medical application requires a predetermined throughput to be maintained in processing a large amount of cells.

A transgenetic method includes a biological approach, such as a vector method, a chemical approach, such as a transfection, and a physical approach, such as an electroporation, a particle gun and an injection. The biological and chemical approaches are not suitable for medical applications because they limit combinations between cells and introduced materials. On the other hand, the physical approach is known as a method that does not limit the combinations. In particular, the injection approach (see, for example, Japanese Patent Applications, Publication Nos. 9-187278 and 2000-23657) has a high introduction success rate as widely used for artificial inseminations, and is likely to be adopted as a next-generation transgenetic method. According to the prior art injection approach, a skilled operator uses a microscope to introduce material from a needle tip into a cell while minimizing damage to the cell. Since the injection becomes difficult when the cells are allowed to freely move on a laboratory dish, a method has been proposed of attracting and fixing multiple cells at the same time via a porous membrane and the back of the filter (see, for example, Japanese Patent Application, Publication No. 2-117380).

Other prior art include, for example, Japanese Patent Application, Publication No. 2004-166653 and Japanese Patent No. 3,525,837.

When a cell is attracted or absorbed, a cell that is a sphere before the attraction deforms to a flat shape. In addition, when a cell is cultivated on a laboratory dish in a floating state without attraction, cells divide, spread over and adhere to the dish surface. In this case, each cell also deforms from a sphere to a flat shape. In the meantime, some materials, such as DNA, which can be inserted into any region in a cell, and others, such as medication and protein, need to be inserted into a specific region in the cell, such as a cell nucleus or a cytoplasm.

In this case, a manual injection of medication has a low throughput. Even a skilled operator can inject the medication into a limited number of cells per unit hour. Manual injection thus requires a lot of time to obtain the necessary number of cells. It would be preferable if an injection apparatus could automatically inject medication etc. into cells so as to improve the operational throughput.

A conventional injection apparatus positions a capillary by setting a target position a predetermined distance above an attraction port or dish surface, and does not have any means for inserting a material into a specific region. In a flatly deformed cell, a specific region may be so thin depending upon the capillary's insertion direction that an insertion becomes difficult, for example, the capillary pierces the specific region. Moreover, depending upon a type of the cell and an insertion angle of the capillary, the surface resistance of the cell is so high that a tip of the capillary is repelled by or slips on the cell surface. Given the visual inspection by the skilled operator, the insertion position of the capillary would be properly determined, but this is not preferable in view of the throughput as described above.

The present invention is directed to an injection apparatus and method, which can precisely inject a material into a specific region in a minute object with a high throughput.

An injection apparatus according to one aspect of the present invention that inserts an injection needle into a target region in a minute object, and injects a predetermined material into the target region from the injection needle includes a positioning controller that determines, as an insertion direction, a direction that provides the longest length in the target region among plural directions in each of which the injection needle can be inserted into the minute object, and a moving unit that moves the injection needle along the insertion direction. According to the injection apparatus, the positioning controller sets a direction that provides a largest margin to the insertion position, stabilizing the insertion of the injection needle and an injection of the material.

The plural directions may be parallel or non-parallel to each other. The positioning controller preferably determines, as an insertion position of the injection needle, a midpoint of a line segment in the target region along the insertion direction, the moving unit moving the injection needle to the insertion position. The midpoint provides the largest margin to the insertion position. A measuring unit that measures a shape of the minute object uses, for example, an optical cutting (scanning or sectioning) method that scans the minute object with measuring light, such as a laser beam, and obtains an image of the locus using an image taking unit, such as CCD, or a microscopic optical system with a confocal X-direction scanning microscope. Preferably, an absolute value of an angle between each of the plural directions and a normal to a surface of the minute object is a predetermined angle or smaller. This configuration can exclude a direction in which the injection needle would be repelled or slipped on the surface of the minute object, from the insertion-direction candidates.

An injection apparatus according to another aspect of the present invention that inserts an injection needle into a target region in a minute object, and injects a predetermined material into the target region from the injection needle includes a positioning controller that determines, as an insertion direction, a direction that provides a length equal to or greater than a predetermined length in the target region and is closest to a normal to a surface of the minute object, among plural directions in each of which the injection needle can be inserted into the minute object, and a moving unit that moves the injection needle along the insertion direction. This injection apparatus addresses the insertion angle makes the insertion angle close to the normal to the surface of the fine object, as long as the insertion position can secure a margin to some extent, preventing the injection needle from being repelled by, or slipping on the surface of the minute object.

The minute object may have a first part as the target region, and a second part different from the first part, wherein when a pair of first parts exists at both sides of the second part along the insertion direction, the length in the target region is a longer one of lengths of the pair of first parts or a closer one of the lengths of the pair of first parts to the injection needle. The longer length secures the margin for the insertion position, and the closer length avoids interference with the second part.

An injection apparatus that inserts an injection needle into a target region in a minute object, and injects a predetermined material into the target region from the injection needle includes a positioning controller that determines, as an insertion position of the injection needle in the minute object, a center of the largest inscribed sphere in the target region, and a moving unit that moves the injection needle to the insertion position. This injection apparatus secures the insertion-position margin using a sphere. The positioning controller may determine, as an insertion direction, a direction that is closest to a normal to a surface of the minute object, among directions in which the injection needle can be moved to the insertion position, the moving unit moving the injection needle along the insertion direction. This configuration can exclude a direction in which the injection needle is repelled or slipped on the surface of the minute object, from the insertion-direction candidates. The positioning controller may select the insertion direction among the directions in which the injection needle can be moved to the insertion position, and among directions that provide a projected area of the minute object smaller than a predetermined area. When the projected area is large, the minute object spreads out in a direction orthogonal to a direction of the wide projected area. When a small projected area is selected, the margin of the insertion position increases.

Preferably, the minute object includes a first portion as the target region, and a second portion different from the first portion, wherein the plural directions do not include a direction that crosses the first area. Thereby, the second part is prevented from getting damaged by the injection needle.

Preferably, the injection apparatus further includes a container that accommodates the minute object, wherein at least one of the moving unit and the container has an adjuster that changes an arrangement and an orientation between the injection needle and the minute object. The arrangement is expressed, for example, by positions in XYZ directions, and the orientation is expressed, for example, by angles around each of XYZ directions.

An injection method according to another aspect of the present invention that inserts an injection needle into a target region in a minute object, and injects a predetermined material into the target region from the injection needle includes the step of determining, as an insertion direction of the injection needle, one of plural directions in each of which the injection needle can be inserted into the minute object, based on at least one of a length in the target region along each of the plural directions, an angle between each of the plural directions and a surface of the minute object, and a projected area of the minute object, or the step of determining, as an insertion position of the injection needle in the minute object, a center of the largest inscribed sphere in the target region. This injection method can secure the margin for the insertion position by taking into account the length in the target region along the insertion direction, an angle between the insertion direction and the surface of the minute object, a projected area of the minute object, and the largest inscribed sphere in the target region.

Further aspects of the present invention provide various manufacturing methods for a minute object into which material has been injected as set out in the accompanying claims.

Other features of the present invention will become readily apparent from the following description of the preferred embodiments with reference to accompanying drawings, in which:
FIG. 1 is a schematic sectional view of an injection apparatus according to one embodiment of the present invention.
FIG. 2 is a schematic sectional view for explaining an operation of the injection apparatus shown in FIG. 1.
FIG. 3 is a schematic sectional view for explaining a calculating principle of an inserting direction and an inserting position for the cell shown in FIG. 2.
FIG. 4 is a flowchart for explaining a calculation of the insertion direction and the insertion position shown in FIG. 3.
FIG. 5A is a schematic sectional view for explaining a calculation of the insertion direction and the insertion position by a detector and a main controller shown in FIG. 2. FIG. 5B is a flowchart for explaining an operation of a configuration shown in FIG. 5A.
FIG. 6 is a schematic view of an optical cutting method that measures a shape of the cell shown in FIG. 2.
FIG. 7 is an optical-path diagram showing a confocal optical system that measures a shape of the cell shown in FIG. 2.
FIG. 8 is a schematic sectional view for explaining a calculation principle of the insertion direction and the insertion position when a target region in the cell shown in FIG. 2 is a cytoplasm.
FIG. 9 is a flowchart for explaining a calculation of the insertion direction and the insertion position shown in FIG. 8.
FIG. 10 is a schematic sectional view for explaining a principle that corrects the insertion direction and the insertion position shown in FIG. 8 by taking into account an angular difference between an insertable direction into the cell and a normal to the surface of the cell.
FIG. 11 is a schematic sectional view for explaining a variation of the principle shown in FIG. 8 which determines the insertion direction after determining the insertion position.
FIG. 12 is a schematic sectional view for explaining a principle that determines the insertion direction from a relationship between an insertable direction into the cell and a projected area.
FIG. 13 is a view for explaining an injection using the injection apparatus shown FIG. 1.
FIGs. 14A and 14B are plane and sectional views for explaining the injection using the injection apparatus shown in FIG. 1.
FIG. 15 is a flowchart for explaining the injection operation using the injection apparatus shown in FIG. 1.

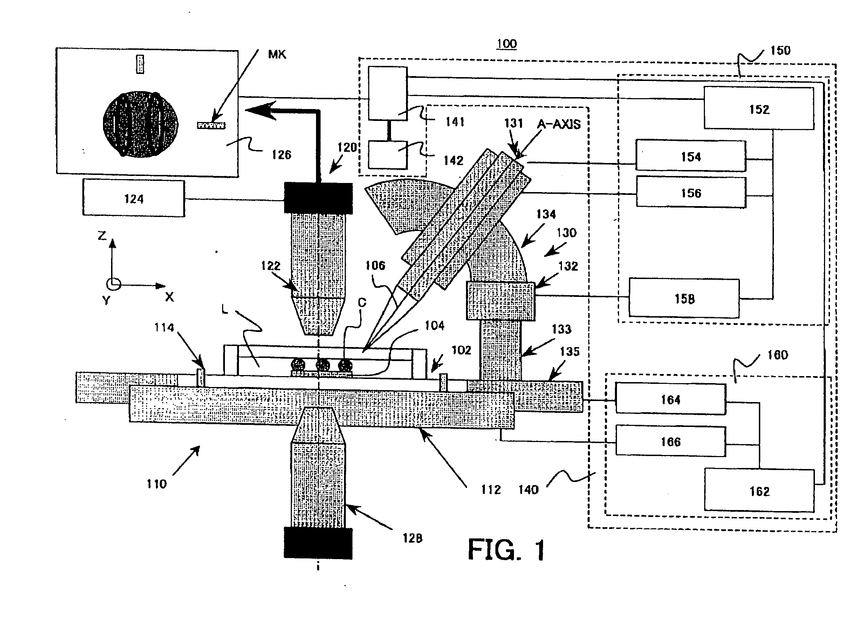

A description will now be given of an injection apparatus 100 according to one embodiment of the present invention, with reference to the accompanying drawings. The injection apparatus 100 injects a predetermined material into a minute object applicable to the present invention, such as a cell and a colloid, using a capillary. The minute object spreads and can float in a fluid or liquid L The fluid L is, for example, cell suspension and medium. Here, FIG. 1 is a schematic sectional view of the injection apparatus 100. The injection apparatus 100 includes, as shown in FIG. 1, a laboratory dish 102, a capturing base 104, a capillary 106, a dish support system 110, an observation system 120, a capillary driving system 130, and a control system 140.

The dish 102 is a container in light gray in FIG. 1 that houses the capturing base 104, the fluid L, and plural cells C. The dish 102 is a cylindrical liquid bath that houses the fluid L and the plural cells C that float in the fluid L, and made, for example, of glass or plastic. The dish 102 is replaced with a container that has a channel in another embodiment. The dish 102 is connected to one or more supply parts (not shown) that supply the cells C and the fluid L.

An amount of the fluid L is such that the cells C float in the fluid L without contacting the capturing base 104 during a normal time period and the cells C are restricted on the capturing base 104 during an attraction time period. Since the fluid L flows in the capturing base 104 from attraction ports (not shown), which will be described later, a sufficient amount of the fluid L needs to be supplied to the dish 102 so that the cells C can float without contacting the capturing base 104 even after the fluid L is absorbed in the capturing base 104.

The dish 102 is provided with a sensor that detects a height of the fluid L. The dish 102 may be configured to receive the fluid L from the supply part (not shown) when the sensor detects that the fluid amount is lower than a predetermined height. The dish 102 may be connected to a channel that feeds the cells into which a predetermined material has been injected (or processed cells) to a subsequent recovery part.

The capturing base 104 is a cylindrical member that fits an inner surface of the dish 102, and captures the cells C. The capturing base 104 is made of a material, such as glass and plastic, which has a specific gravity greater than the fluid L and the cells C and does not contaminate the fluid L. The capturing base 104 may be part of the dish 102.

The capturing base 104 has plural attraction ports (not shown), and a decompression unit connected to them. While this embodiment includes the capturing base 104, the cells C adhere to the dish surface when dividing, and thus it is optional to provide the capturing base 104.

Each attraction port is provided in a surface facing the cells C, and is a hole with a diameter of several micrometers. Each attraction port attracts and captures the cell C. The number of attraction ports is set to the maximum number of the cells to be captured simultaneously. The attraction port can attract and exhaust the cell C, exhaust the fluid L, and is connected to the decompression unit, such as a pump. The number of pumps may be the sane as the number of attraction parts. When each attraction port is provided with a control valve, at least one pump is sufficient.

The decompression unit may use any type as long as at least it provides the attraction (and exhaustion preferably). For example, it may be an attraction/exhaustion apparatus that includes a cylinder and a plunger that can slide in an axial direction of the cylinder. This embodiment allows the attraction and exhaustion forces to be adjustable. The attraction force used to capture the cell C without damaging it, and the exhaustion force that releases the processed cell C are previously set by simulation or experimental results. The attraction force used to draw the cell to the attraction port, and the exhaustion force of the fluid L used to disperse the agglutinating cells C are adjustable, thereby improving the capturing efficiency.

The capillary 106 is an injection needle that is inserted into a specific region in the cell C, such as a nucleus and a cytoplasm, and injects predetermined material, such as medication and protein, to the specific region. The capillary 106 is a hollow member made, for example, of glass and plastic, and connected to and driven by a capillary driving system 130. At least one of the dish supporting system 110 that fixes and supports the dish 102 and the capillary driving system 130 has an adjuster that changes an arrangement and orientation between the capillary 106 and the cells C.

The arrangement position is, for example, positions in XYZ directions, and the orientation is, for example, one or more angles around each of the XYZ-axes. In this embodiment, an XY table 112 of the dish supporting system 110 serves to move the dish 102 on an XY plane, and the capillary driving system 130 has a moving or fine adjustment function in other directions.

The dish supporting system 110 serves as a means that horizontally fixes and supports the dish 102, and includes an XY table 112, and plural positioning pins 114.

The XY table 112 has a horizontally maintained and fixed, cylindrical shape and roughly positioned on the XY plane by an XY controller 166. The capillary driving system 130 provides fine adjustments of the capillary 106 and the cells C.

The positioning pins 114 fix and position the dish 102 on the XY table. The positioning pins 114 are arranged at a regular interval, but the number of them is not limited. While this embodiment fixes the dish 102, the dish 102 may be displaceable as discussed above in the XYZ-axes directions and around these axes (or in aβγ-axes directions), as discussed above.

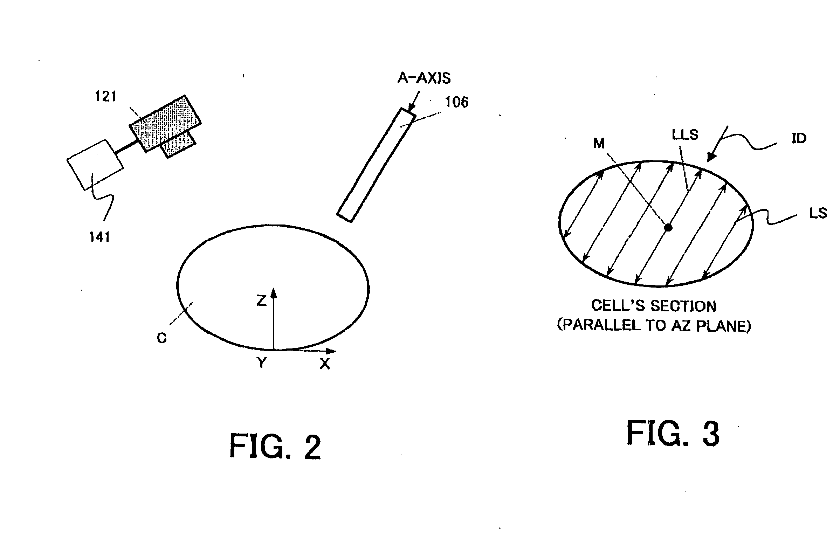

The observation system 120 includes a measuring unit and a status observing unit. The measuring unit measures a shape of the cell C. The status observing unit observes whether the capillary 106 reaches a target position in the cell C, and whether the injection of the material has ended. The measuring unit includes a cell orientation acquiring optical system 122, an image processor 124, and an image taking unit 126. It is implemented as a measuring unit 121 in FIG. 2 and subsequent drawings. The image taking unit 126 includes a CCD, etc., and a reference numeral 126 in FIG. 1 denotes a screen displaying an image taken by the CCD. The status observing unit includes an injection status observing optical system 128.

The capillary driving system 130 has a moving unit that moves the capillary 106 in a set direction (which is referred to as a "A-axis direction" or "insertion direction" in this embodiment), and an adjuster that changes an arrangement and orientation between the capillary 106 and the cell C. In an illustration in FIG. 1, the A-axis direction accords with an axial direction of the capillary 106.

The moving unit includes a capillary insertion/ejection direct-acting table (A-axis table) 131 that is coupled with the capillary 106 and moves the capillary 106 in the A-axis direction.

The adjuster includes capillary XY-axes 132, a capillary Z-axis 133, a β-axis table 134, and a γ-axis table 135. The capillary XY-axes 132 provide fine adjustments for the capillary 106 in the XY directions. The capillary Z-axis 133 moves the capillary 106 in the Z-axis direction. The β-axis table 134 rotates the capillary 106 around the Y-axis or in "B-axis" direction. The γ-axis table 135 rotates the capillary 106 around the Z-axis or in "γ-axis" direction. The γ-axis table 135 is provided around the dish 102, and has, for example, an annular shape. FIG. 1 omits a driving mechanism around the X-axis or in "a-axis" direction.

The control system 140 includes a main controller 141 that calculates an insertion direction and an insertion position of the capillary 106, a memory 142, a capillary control system 150 that controls driving of the capillary 106, and a table control system 160 that controls operations of tables.

The main controller 141 calculates the insertion direction and the insertion position in the cell C used to insert the capillary 106, based on a measurement result by the measuring unit of the observation system 120. Next, the main controller 141 controls the capillary control system 150 and the table control system 160 based on the calculated insertion direction and insertion position. The main controller 141 controls the capillary control system 150 based on an injection status result from the status observing unit in the observation system 120. The main controller 141 controls various components of the injection apparatus 100, such as the attraction/exhaustion part of the cell C, the supply part of the fluid L, and the supply part of the material.

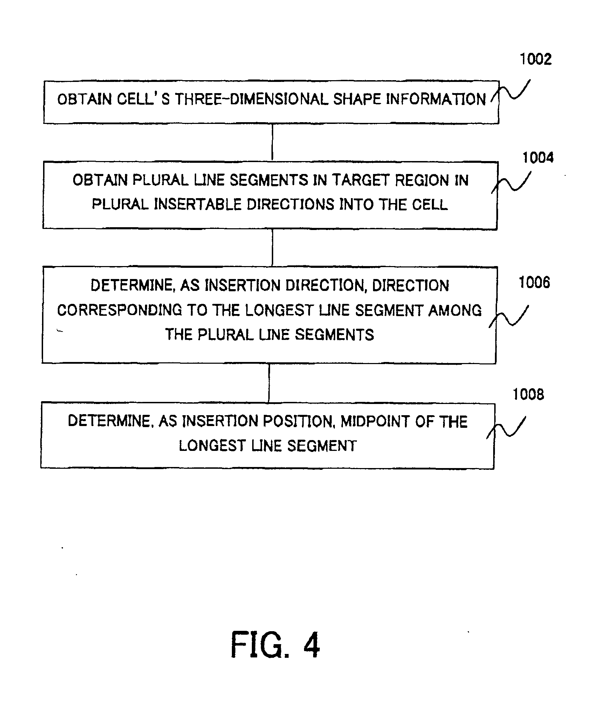

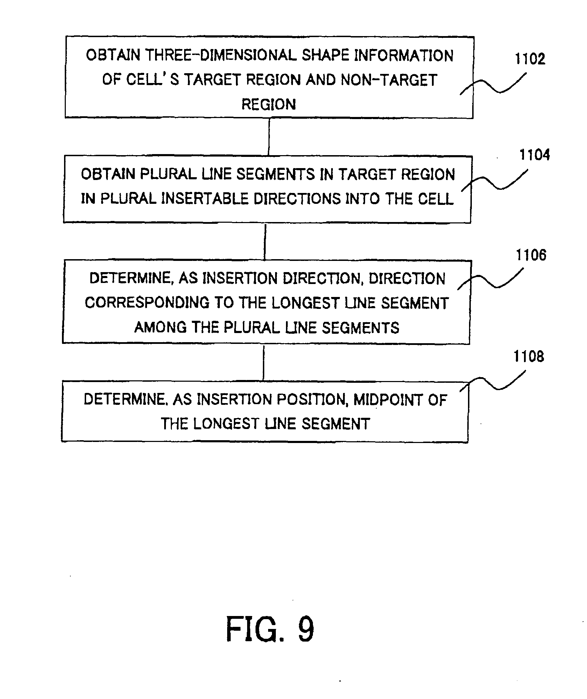

The memory 142 stores operational methods of the main controller 141, such as flowcharts shown in FIGs. 4 and 9, and various data.

The capillary control system 150 includes a capillary operation controller 152, an insertion/ejection controller 154, a β controller 156, and an XYZ controller 158. The capillary operation controller 152 is controlled by the main controller 141, and controls the insertion/ejection controller 154, the β controller 156, and the XYZ controller 158 under control of the main controller 141. The insertion/ejection controller 154 controls movements of the capillary 106 in the A-axis direction by the capillary insertion/ejection direct-acting table 131. The β controller 156 controls an inclination of the capillary 106in the β-axis direction by the β-axis table 134. The XYZ controller 158 provides fine adjustments to the movements of the capillary 106 by the capillary XY-axes 132 and the capillary Z-axis 133 in the XYZ directions.

The table control system 160 includes a table operation controller 162, a γ controller 164, and an XY controller 166. The table operation controller 162 is controlled by the main controller 141, and controls the γ controller 164 and the XY controller 166 under control of the main controller 141. The γ controller 164 controls a rotating angle of the capillary 106 in the γ direction by the γ-axis table 135. The XY controller 166 controls movements of the XY table 112 on the XY plane.

Referring now to FIGS. 2 to 4, a description will be given of the principle by which the main controller 141 calculates the insertion position. Here, FIG. 2 is a schematic sectional view showing an insertion of the capillary 106 into the cell C by fixing the insertion direction of the capillary 106. FIG. 3 is a schematic sectional view for explaining a calculation of the insertion position in the cell C. FIG. 4 is a flowchart for explaining an operation of the main controller 141. FIGs. 2 and 3 assume that the target region is the whole of the cell C. In addition, FIG. 2 assumes that the capillary 106 can be inserted into the cell C along the A-axis or an axis parallel to it. A reference numeral 121 is a measuring unit or a camera, and corresponds to a combination of components 122 to 126 in FIG. 1.

In FIG. 2, the measuring unit 121 measures a stereoscopic or three-dimensional shape of the cell C fixed on the XY plane, and the main controller 141 obtains the measurement result of the measuring unit 121. As a result, the main controller 141 obtains the (external) shape of the cell C (step 1002).

Next, the main controller 141 recognizes directions in which the capillary 106 can be inserted, as shown by arrows in FIG. 3, and obtains lengths of line segments LS in the cell C in respective directions (step 1004). Each line segment LS qualifies the insertion direction of the capillary 106. FIG. 3 is a schematic sectional view of the cell C on the plane that contains the A-axis and Z-axis. In an illustration in FIG. 3, the main controller 141 obtains lengths of line segments in the cell C at plural positions parallel to the specific direction (linear direction in FIG. 3). An interval between line segments and the number of line segments may be arbitrarily determined. While FIG. 3 sets line segments along parallel plural lines, line segments may be set along plural unparallel directions and their lengths may be obtained.

Next, the main controller 141 selects the longest line segment LLS among the plural line segments LS, and determines a direction along the segment LLS as an insertion direction ID (step 1006). The insertion direction ID of the capillary 106 does not have to perfectly accord with the segment LLS. With a permissible offset, once a vicinity of the line segment LLS is selected, the insertion position or direction may be shifted. A permissible shift amount is appropriately set on a case-by-case basis. As discussed above, the cell C may deform to a flat shape, when it is attracted or adheres to the dish surface. Since the main controller 141setsas the insertion direction a direction that provides the largest margin to an insertion position, an insertion of the capillary 106 and a material injection are made easier.

Next, the main controller 141 selects as an insertion position a midpoint M of the line segment LLS (step 1008). On the line segment LLS, the midpoint M provides the largest margin for both the front and back of the midpoint. In FIG. 3, the insertion position is a position at which a tip of the capillary 106 is to be arranged. The capillary operation controller 152 controls the adjuster and moves the capillary so that the tip of the capillary 106 reaches the insertion position. The present invention does not intend to limit the insertion position to the midpoint M, and allows a shift from the midpoint M as long as the shifted position is located on the line segment LLS. An alternative embodiment does not consider the insertion position of the capillary 106.

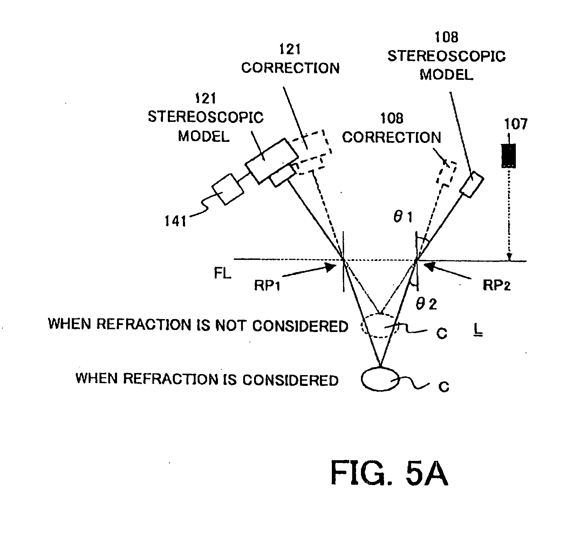

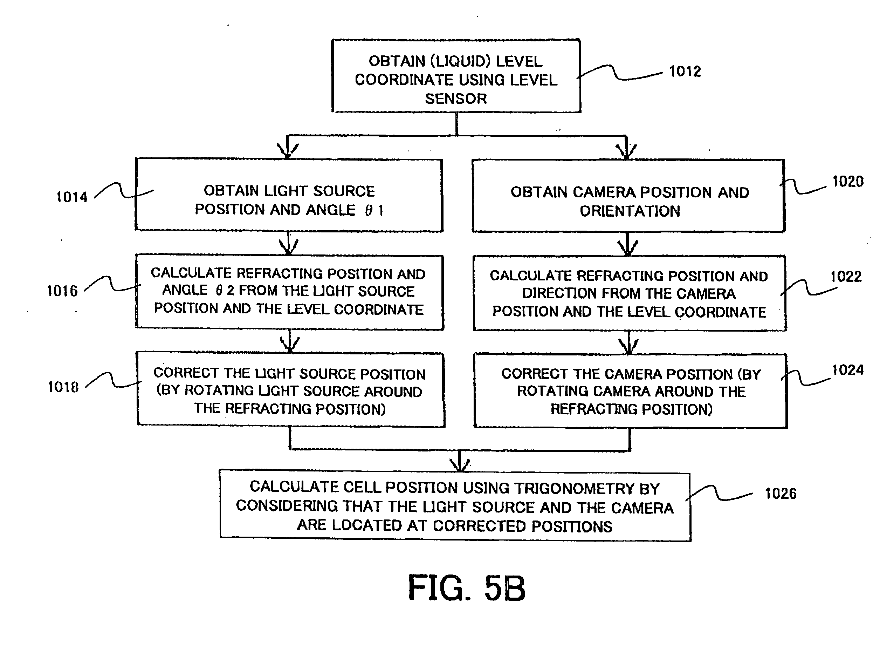

As described above, the cells C float in the fluid L. Therefore, it is preferable as shown in FIG. 5A that the measuring unit 121 and the main controller 141 consider the refractive index of the fluid L and measure the shape of the cell C. When the refractive index of the fluid L is not considered, the apparent position of the cell C differs from the actual position of the cell C in the liquid L, and the insertion direction of the capillary C may possibly shift. Here, FIG. 5A is a schematic sectional view showing a shape of the cell C when the measuring unit 121 and the main controller 141 consider refraction in measuring the shape of the cell C. FIG. 5B is a flowchart for explaining an operation of the configuration shown in FIG. 5A.

In FIG. 5A, the controller 141 initially obtains a coordinate of the surface of the fluid FL using a (liquid) level sensor 107 (step 1012).

Next, the controller 141 obtains a position and an inclination θ1 of the light source 108 (step 1014) (step 1014). Next, the controller 141 calculates a refracting position RP2 and the direction θ2 from the coordinates of the light source 108 and the surface FL (step 1016). Next, the controller 141 corrects a solid line position to a broken line position shown in FIG. 5A by rotating the position of the light source 108 around the refracting position RP2 (step 1018).

The controller 141 obtains a position and an inclination of the camera 121 (step 1020). Next, the controller 141 calculates a refracting position RP1 and the direction from the coordinates of the camera 121 and the surface FL (step 1022). Next, the controller 141 corrects a solid line position to a broken line position shown in FIG. 5A by rotating the position of the camera 121 around the refracting position RP1 (step 1024).

It does not matter which of steps 1014-1018 and steps 1020-1024 are performed first. Finally, the controller 141 calculates the position of the cell C using the trigonometry by considering that the light source 108 and the camera 121 are located at the broken-line corrected positions shown in FIG. 5A.

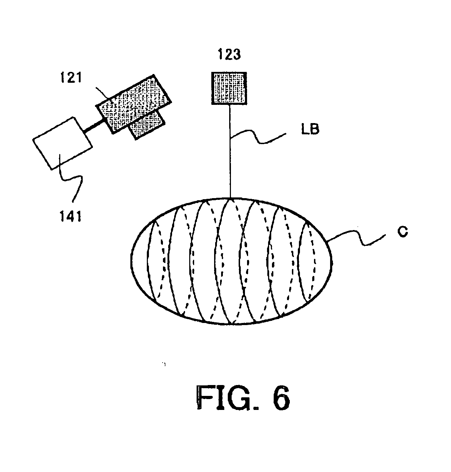

Referring now to FIG. 6, a description will be given of a shape measuring method of the cell C using the measuring unit 121. While this embodiment uses the shape of the cell C using the optical approach, the present invention may use a sound wave (ultrasound) etc.

Here, FIG. 6 is a schematic view for explaining the principle of the optical cutting method for measuring the shape of the cell C. The measuring unit 121 measures the dispersion and reflected light that occur when a laser light source 123 scans the cell C with a laser beam LB, thereby measuring the shape of the cell C three-dimensionally. The main controller 141 considers the reflection and refraction that occur on the surface FL of the fluid L, and calculates the position of the surface FL of the fluid L in addition to the position and (external) shape of the cell C.

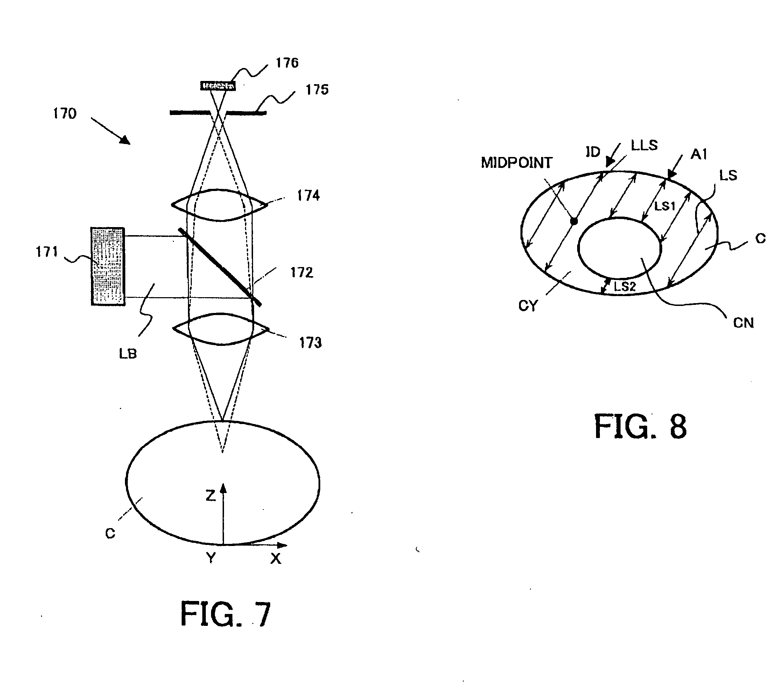

An alternative embodiment measures the shape of the cell C by utilizing a stereoscopic optical system that includes a confocal Z-direction scanning microscope. Here, FIG. 7 is a schematic optical-path diagram of the confocal optical system 170. The confocal optical system 170 includes a laser light source 171, a half mirror 172, an objective lens 173, a condenser lens 174, a pinhole 175, and a light-receiving element 176. The confocal optical system 170 is an optical system that eliminates the light other than a focus position of the objective lens by arranging the pinhole 175 in front of the light-receiving element 176. The light receiving amount at the focus position is extremely large in the confocal optical system 170, and a variation amount of the focal position can be detected based on a light-receiving amount. The shape of the cell C is measured based on the variation amount of the focus position.

In FIG. 7, a solid line denotes a focus state, and a broken line denotes a defocus state. In the focus state, the laser beam LB from the laser light source 171 is reflected on the half mirror 172, and condenses on the cell C via the objective lens 173. Most of the reflected light is received by the light-receiving element 176 via the objective lens 173, half mirror 172, condenser lens 174, and the pinhole 175. On the other hand, in the defocus state, only part of the reflected light is incident upon the light-receiving element 176.

The confocal Z-direction scanning microscope that applies the stereoscopic optical system 170 combines this optical system with a fast XY scanner, and obtains a high-resolution focus image and the shape of the cell C. The stereoscopic shape of the cell C is measured by utilizing the confocal optical system 170 that detects the dispersion and reflected light from a beam spot irradiated onto the cell C through a pinhole or slit 175 at an imaging surface of the microscopic optical system, and by moving the focal point in the Z direction. The fast XY scanner includes a resonant scanner and a galvano-scanner. A lateral fast scan uses the resonant scanner, and a longitudinal scan requires a positioning accuracy and uses the galvano-scanner. The light-receiving element 176 can include, e.g. a photomultiplier (PMT). Similar to FIG. 6, the main controller 141 can calculate the accurate shape and position of the cell C by considering the refractive index of the fluid L.

While the above embodiment describes two approaches, other approaches are applicable as long as they can detect the interface of the cell C.

FIG. 3 assumes that the target region is the whole of the cell C. However, the cell C includes a cell nucleus CN and a cytoplasm CY as shown in FIG. 8, and an actual application injects a predetermined material, such as medication, into only one of them. In this situation, it is preferable to determine the capillary's insertion direction and insertion position by particularly considering the shape measuring result of the cell nucleus CN. Referring now to FIG. 9, a description will be given of an operation of the main controller 141 when the target region is the cell nucleus CY.

FIG. 8 is a schematic sectional view for explaining an operation that determines an insertion position of the capillary 106 when the cell nucleus CN is the target region in the cell. FIG. 9 is a flowchart for explaining an operation of the main controller 141.

First, the measuring unit 121 measures stereoscopic shapes of the target region (cell nucleus CN) and the non-target region (cytoplasm CY) in the cell C fixed on the **XY plane.** The main controller 141 obtains the measurement result of the measuring unit 121. As a result, the main controller 141 obtains the (external) shape information of the cell C and the shape information of the cell nucleus CN (step 1102). The shape information can contain shapes, sizes, and positions of the cell C and the cell nucleus CN.

Next, the main controller 141 recognizes directions in which the capillary 106 can be inserted as shown by arrows shown in FIG. 8, and obtains lengths of line segments LS in the cell nucleus CN in each direction (step 1104). While FIG. 8 sets parallel line segments, line segments in plural unparallel directions may be set and their lengths may be obtained.

The main controller 141 may exclude a direction that crosses the cell nucleus CN among plural directions shown in FIG. 8. Thereby, in injecting the material into the cell nucleus CN, the cell nucleus CN is prevented from getting damaged by the capillary 106. In this case, the longest line segment (LLS shown) is selected as an insertion direction for the capillary 106 among the line segments that do not pass the cell nucleus CN. As discussed above, the midpoint of the segment LIS is set to the insertion position for the capillary 106, securing the largest margin for inserting the capillary 106.

On the other hand, in injecting the medication into the cell nucleus CN, lengths of line segments that cross the cell nucleus CN are selected. A line that passes the cell nucleus CN provides two line segments LS1 and LS2 in a certain direction A1. The main controller 141 counts the number of crosses between the line that extends in the A1 direction and the cell C/cell nucleus CN, executes a logic difference between the cell C's (external) shape and the cell nucleus CN's shape, and calculates the space of the cytoplasm CY (lengths of the line segments LS1 and LS2). With respect to the direction A1, the main controller 114 selects as the capillary's insertion direction a longer one of the line segments CS1 and CS2 or a line segment that is closer to the capillary 106.

In this embodiment, the line segment LS1 is longer than and closer to the capillary 106 than the line segment LS2. When the margin of the insertion position is addressed, the segment LS1 that has a longer interval in the cytoplasm CY is selected as an insertion direction in the direction A1.

When the capillary 106 is inserted through the cell nucleus CN, the cell nucleus CN gets damaged undesirably. When an insertion into the cytoplasm CY intends to avoid the cell nucleus CN, the line segment closer to the capillary 106 will be selected for the capillary's insertion direction.

In the illustration in FIG. 8, both a longer line segment and a line segment closer to the capillary are LS1. However, if LS1 < LS2 is met regarding the length of the line segment, one of them is selected as the capillary's insertion direction. When the capillary 106 can be rotated by 180° and inserted into the cell C from the bottom in FIG. 8 along a opposite direction to the direction A1, a longer line segment among LS1 and LS2 is selected as an insertion direction for the capillary 106 without selecting a line segment closer to the capillary 106.

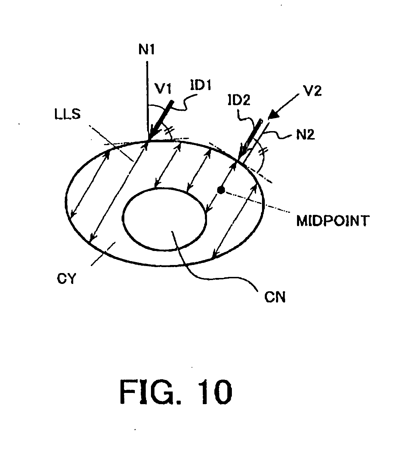

Among the line segments that pass the cell C, the main controller 141 may exclude from candidates for the capillary's insertion direction, as shown in FIG. 10, those line segments which provide an absolute value of an angle equal to or greater than a predetermined angle between the capillary 106's insertion direction and a normal N to the surface of the cell C. A small insertion angle causes the capillary to be repelled from the cell surface due to the elastic force. The above selection enables the capillary 106 to be inserted into the cell C at an angle close to the normal to the cell surface by excluding directions in which the capillary 106 is hard to insert into the surface of the cell C from the insertion-direction candidates.

FIG. 10 is a schematic sectional view for explaining an insertion position when the target region is the cytoplasm CY in the cell C. When the longest line segment is selected in the cytoplasm CY by avoiding the cell nucleus CN in inserting the capillary 106, the ID1 shown is the capillary's insertion direction and a midpoint of the longest line segment LLS is selected as the insertion position. Nevertheless, in FIG. 10, an angle V1 between the normal N1 and the insertion direction ID1 is greater than a predetermined angle, and the capillary 106 is likely to be deflected by the surface of the cell C. Therefore, it is excluded from the insertion-direction candidate. In the illustration in FIG. 10, the main controller 141 selects a direction ID2 as an insertion direction, and a midpoint of the corresponding line segment (black dot shown) is selected as an insertion position of the capillary 106, since a difference V2 is smallest between the normal N2 to the cell surface and the capillary's insertion angle (or closest to the perpendicular to the surface of the cell C) in the direction ID2.

The main controller 141 in the alternative embodiment adopts a slightly greater threshold for a larger angular difference between the normal and the insertable direction. A user of the injection apparatus 100 can set the threshold of the angular difference depending upon the type of the cell C.

The main controller 141 may determine, as the insertion direction, one of directions in which the capillary 106 can be inserted into the cell C, when the one provides a length that passes cytoplasm CY, is greater than a predetermined threshold, and is closest to the normal of the surface of the cell C. As long as it maintains a certain margin for an insertion position, the capillary 106 is prevented from being repelled by making the inserting angle close to the normal.

Next, the main controller selects, similar to the step 1006, the longest line segment LLS among plural line segments LS, and determines a corresponding direction as the insertion direction ID (step 1106). As discussed above, the cell C may deform to a flat shape, when it is attracted or adheres to the dish surface. Since the main controller 141 sets to the insertion direction a direction that provides the largest margin for an insertion position, an insertion of the capillary 106 and an injection of the material are stable.

Next, the main controller 141 selects as an insertion position a midpoint M of the line segment LLS (step 1108), because the midpoint M provides the largest margin for the insertion position. In FIG. 8, the insertion position is a position at which a tip of the capillary 106 is to be arranged. The present invention does not intend to limit the insertion position to the midpoint M, and allows a shift from the midpoint M as long as the shifted position is located on the line segment LLS.

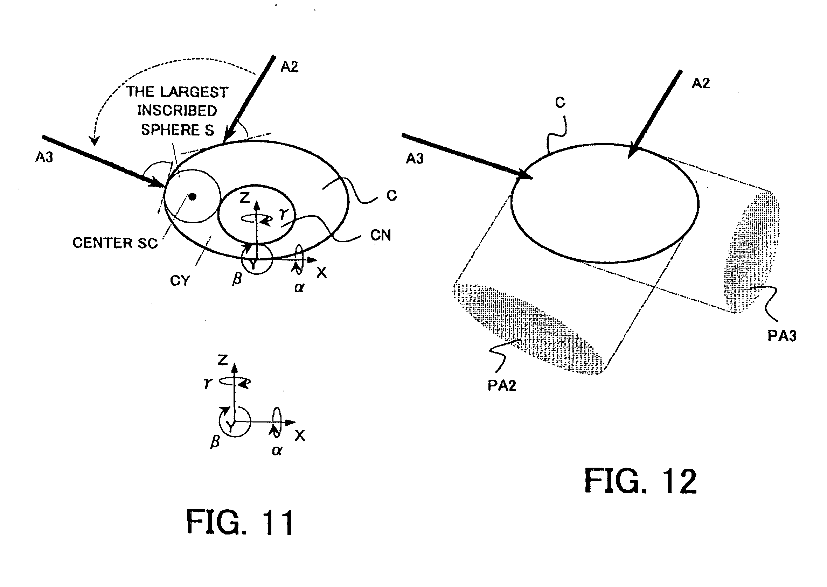

In the above embodiment, the main controller 141 sets the insertion direction, and then sets the insertion position. However, as shown in FIG. 11, the insertion position may be set first. Here, FIG. 11 is a schematic sectional view for explaining a method for setting the insertion direction after setting the insertion position.

In FIG. 11, the main controller 141 determines, as the insertion position for the capillary 106, a center SC of a sphere S having a maximum diameter inscribed in the cytoplasm CY when the target region is the cytoplasm CY.

The sphere S included in the cytoplasm CY (or inscribed in a space between the cell C surface and the cell nucleus CN or only in the cell C) is calculated from shape information of the cell C and the cell nucleus CN. An interval between the cell C's surface and the cell nucleus CN based on the shape information, or an interval between the cell C's surface that do not interpose the cell nucleus CN is calculated. The interval is calculated with respect to plural directions, and the inscribed sphere in the cell C or between the cell C and the cell nucleus CN and its diameter are calculated. After these plural spheres are calculated, the sphere having the largest diameter is selected among them and the center SC is set to the insertion position of the capillary 106.

Another method may be used to calculate the sphere. While this embodiment uses a sphere, a solid having another shape may be properly set to determine the insertion position of the capillary 106.

The interval between the cell C's surface and the cell nucleus CN maintains at least the diameter of the sphere S, and the margin of the insertion position is secured using the sphere. Next, the main controller 141 determines, as the insertion direction, a direction of the capillary's insertion direction that is close to the normal to the cell C's surface, excluding a direction in which the capillary 106 would be deflected from the cell C's surface, from the insertion-direction candidates.

In **FIG.** 11, the main controller 141 controls the position and orientation of the capillary 106 by using as an insertion direction a vector that passes the center SC and crosses the cell C's surface at an angle close to a right angle, and by moving the vector in the XYZ-axes and aβγ-axes. As described above, the a-axis is a rotating axis (axis of rotation) around the X-axis. The β-axis is a rotating axis around the Y-axis. The γ-axis is a rotating axis around the Z-axis. Finally, the main controller 141 selects, as the insertion direction of the capillary 106, a direction A3 that provides the insertion angle of the capillary 106 close to a normal to the cell C's surface.

As shown in FIG. 12, the main controller 141 may select, as the insertion direction, one of the directions in which the capillary 106 can be moved to the insertion position, when the one provides a projected area of the cell C smaller than a predetermined area. Here, FIG.12 is a schematic sectional view for explaining an insertion-direction setting method based on a projected area. In FIG. 12, a projected area PA2 of the cell C viewed from a direction A2 is greater than a projected area PA3 of the cell C viewed from a direction A3. When the projected area is large, the fine object spreads out in that direction. Therefore, the cell C spreads out in a direction perpendicular to the direction A2, and the cell C has a small thickness in the direction A2. When a direction that provides a smaller projected area, for example, the direction A3 in the illustration in FIG. 12, is selected as the insertion direction, the margin for the capillary's insertion position increases. Thus, the main controller 141 in this embodiment selects the direction A3. Of course, even in this case, another direction may be selected if the direction A3 crosses the cell nucleus CN or provides a small length of the line segment to the cytoplasm CY. ,

The method in FIG. 12 does not require an individual calculation of each length of the line segment that passes each part in the cell, and can relatively easily determine the insertion direction of the capillary 106.

While this embodiment individually describes plural approaches that determine an insertion position and an insertion direction, they may be combined to determine the insertion position and the insertion by considering required conditions in these approaches as a whole.

For example, FIG. 11 selects the direction A3 as the capillary's insertion direction. However, the cell nucleus CN is located on an extension of the direction A3 and thus can be damaged if the capillary 106 reaches the cell nucleus CN. When avoidance of the damage of the cell nucleus CN is emphasized, a direction that is close to the normal to the cell C's surface may be selected as the insertion direction, for example, in the Y-axis.

Even when the direction A3 is the best only in view of the capillary's insertion angle, another direction may be selected if the other direction is suitable for the capillary's insertion direction in view of other factors. In determining the insertion direction and insertion position of the capillary, which condition should be preferentially treated, how much weight should be imposed, how a threshold used to determine an insertion direction is set, etc. may be appropriately set on a case-by-case basis.

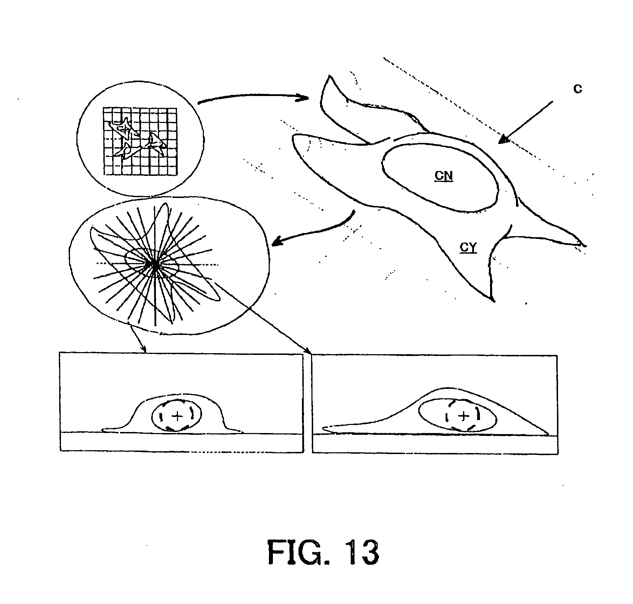

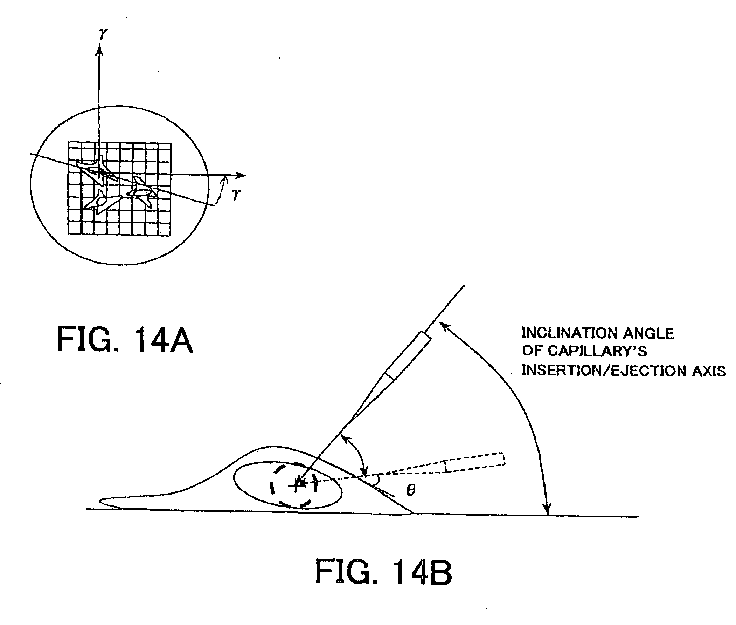

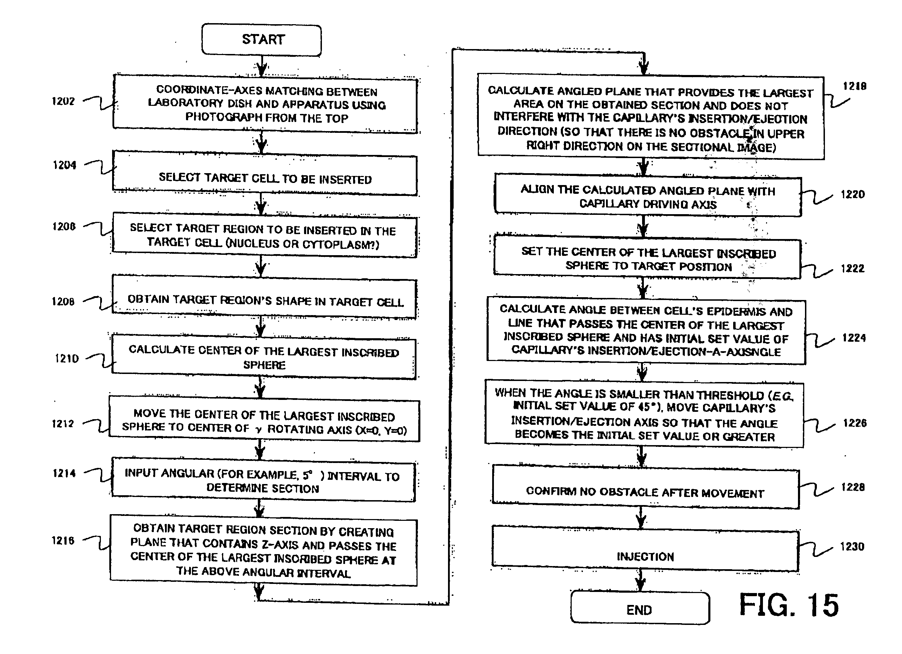

Referring now to FIGs. 13 to 15, a description will be given of a procedure of the material injection from marking MK information (i.e., an orientation detecting pattern on the same field). Here, FIG. 13 is a view for explaining the procedure. FIG. 14A is a plane view for explaining the procedure. FIG. 14B is a sectional view for explaining the procedure. FIG. 15 is a flowchart showing the procedure of the material injection from marking MK information.

U.S. Patent No. 4,907,158 discloses a method of matching a position of the cell C in the dish 102 with a XY coordinate position in the injection apparatus 100 by using the marking MK in the dish 102.

This reference teaches to insert the capillary 106 into a cell C once the cell C is appointed while the dish 102 is horizontally attached and the capillary 106 axis is determined. On the other hand, this embodiment calculates a target position that can secure or allow a tip target position, an insertion angle, and an operational error of the capillary 106. Then, the dish supporting system 110 and the capillary driving system 130 are driven to move the capillary 106 to that target position in the cell C. Thereby, this embodiment improves the injection success rate of the predetermined material into the cell C.

Referring to FIG. 15, the observation system 120 conducts coordinate matching between the dish 102 and the injection apparatus 100 from the image information (step 1202, plane view at the upper left in FIG. 13). The cell C to be inserted is selected (step 1204, enlarge perspective view at the right side in FIG. 13). Steps 1202 and 1204 use a method disclosed in U.S. Patent No. 4,907,158, as discussed above.

Next, a target region in the selected cell C, which is the cell nucleus CN or cytoplasm CY, is selected (step 1206). Here, the nucleus CN is selected. Next, the shape of the target region in the selected cell C is obtained (step 1208). Step 1208 uses, for example, an optical cutting method described in FIG. 6 (enlarged perspective view at the right side in FIG. 13).

Next, a center of the largest inscribed sphere is calculated (step 1210). The center of the largest inscribed sphere is moved to the center of the γ rotating axis (X=0, Y=0) (step 1212). Next, an angular pitch used to select a section is input, such as 5° (step 1214). Next, a plane that contains the Z-axis and passes the center of the largest sphere is obtained as the section for the target region by generating the above angular pitch, as shown by the left center in FIG. 13 (step 1216). This is conducted by converting the measurement result into the three-dimensional data on the computer and by extracting a section from three-dimensional data.

Next, an angled plane is calculated which provides the largest area in the obtained section and the largest area that does not interfere with the capillary's insertion/ejection direction (or has no obstacles on the section image in the upper right direction of the gravity) (step 1218). For example, the sectional area of the target position increases when the lowest right section is selected instead of selecting the lowest left section in FIG. 13. Therefore, an angle γ that provides the right section is obtained. Next, a capillary's driving axis is adjusted to the calculated angled plane (step 1222). In FIG. 14A, the coordinate (X=0, Y=0) of the injection apparatus is adjusted to the center of the largest sphere and rotated by the angle γ. The capillary 106 is located ahead of the X-axis.

Next, an angle θ between the cell skin and the line that passes the center of the largest sphere and corresponds to an initially set value of the capillary's insertion/ejection axis angle is calculated (step 1224, FIG. 14B). When the angle θ is smaller than the threshold (assume that 45° is an initially set value), the capillary's axis is moved so as to maintain the initial set value (step 1226, from a broken line to a solid line in FIG. 14B). Next, after the movement, no obstacles are confirmed (step 1228). Next, the injection follows (step 1230, FIG. 14B).

Further, the present invention is not limited to these preferred embodiments, and various variations and modifications may be made without departing from the scope of the present invention.

Thus, the present invention can provide an injection apparatus and method, which can precisely inject a material into a specific region in a minute object with a high throughput in the injection approach.

## Claims

1. An injection apparatus that inserts an injection needle into a target region in a minute object, and injects a predetermined material into the target region from the injection needle, the injection apparatus comprising:
a positioning controller that determines, as an insertion direction, a direction that provides the longest length in the target region among plural directions in each of which the injection needle can be inserted into the minute object; and
a moving unit that moves the injection needle along the insertion direction.

2. The injection apparatus according to claim 1, wherein the plural directions are parallel to each other.

3. The injection apparatus according to claim 1 or 2, wherein the positioning controller determines, as an insertion position of the injection needle, a midpoint of a line segment in the target region along the insertion direction, the moving unit moving the injection needle to the insertion position.

4. The injection apparatus according to claim 1,2,or 3, further comprising a measuring unit that measures a shape of the minute object using an optical seaming method that scans the minute object with measuring light.

5. The injection apparatus according to claim 1,2,or 3, further comprising a measuring unit that measures a shape of the minute object using a confocal scanning optical system.

6. The injection apparatus according to any preceding claim, wherein an absolute value of an angle between each of the plural directions and a normal to a surface of the minute object is a predetermined angle or smaller.

7. The injection apparatus according to any preceding claim,
wherein the positioning controller determines the insertion direction based on a refractive index of a liquid in which the minute object floats.

8. An injection apparatus that inserts an injection needle into a target region in a minute object, and injects a predetermined material into the target region from the injection needle, the injection apparatus comprising:
a positioning controller that determines, as an insertion direction, a direction that provides a length equal to or greater than a predetermined length in the target region and is closest to a normal to a surface of the minute object, among plural directions in each of which the injection needle can be inserted into the minute object; and
a moving unit that moves the injection needle along the insertion direction.

9. The injection apparatus according to any preceding claim;
wherein the minute object has a first part as the target region, and a second part different from the first part, and
wherein when a pair of first parts exists at both sides of the second part along the insertion direction, the length in the target region is a longer one of lengths of the pair of first parts or a closer one of the lengths of the pair of first parts to the injection needle.

10. An injection apparatus that inserts an injection needle into a target region in a minute object, and injects a predetermined material into the target region from the injection needle, the injection apparatus comprising:
a positioning controller that determines, as an insertion position of the injection needle in the minute object, a center of the largest inscribed sphere in the target region; and
a moving unit that moves the injection needle to the insertion position.

11. The injection apparatus according to claim 10, wherein the positioning controller determines, as an insertion direction, a direction that is closest to a normal to a surface of the minute object, among directions in which the injection needle can be moved to the insertion position, the moving unit moving the injection needle along the insertion direction.

12. The injection apparatus according to claim 11, wherein the positioning controller selects the insertion direction among the directions in which the injection needle can be moved to the insertion position, and among directions that provide a projected area of the minute object smaller than a predetermined area.

13. The injection apparatus according to any preceding claim,
wherein the minute object includes a first portion as the target region, and a second portion different from the first portion, and
wherein the plural directions do not include a direction that crosses the first area.

14. The injection apparatus according to any one of claims 1 to 13, further comprising a container that accommodates the minute object, and
wherein at least one of the moving unit and the container has an adjuster that changes an arrangement and an orientation between the injection needle and the minute object.

15. An injection method that inserts an injection needle into a target region in a minute object, and injects a predetermined material into the target region from the injection needle, the injection method comprising the step of determining, as an insertion direction of the injection needle, one of plural directions in each of which the injection needle can be inserted into the minute object, based on at least one of a length in the target region along each of the plural directions, an angle between each of the plural directions and a surface of the minute object, and a projected area of the minute object.

16. An injection method that inserts an injection needle into a target region in a minute object, and injects a predetermined material into the target region from the injection needle, the injection method comprising the step of determining, as an insertion position of the injection needle in the minute object, a center of the largest inscribed sphere in the target region.

17. An injection apparatus that injects a material into an object, the injection apparatus comprising:
an injecting part that is inserted into the object, and injects the material into the object;
a moving unit that moves the injecting part;
an image taking part that takes an image of the object;
a determining part that obtains a shape of the object taken, and determines an insertion direction of the injecting part based on information relating to the shape of the object;
a controller that controls the moving unit, and moves the injecting part in the insertion direction determined by the determining part.

18. The injection apparatus according to claim 17, wherein the determining part obtains a length and direction of a line segment of the object, and determines the insertion direction based on the line segment.

19. The injection apparatus according to claim 18, wherein the determining part determines the insertion direction based on the longest line segment among plural line segments in the object.

20. The injection apparatus according to claim 17, wherein the determining part determines the insertion direction based on a shape of a first part in the object and a shape of a second part in the first part.

21. A manufacturing method of a minute object into which a material has been injected, the manufacturing method comprising the steps of:
obtaining a length of a line segment in the minute object;
determining an insertion direction for an injection part that injects the material into the minute object, based on the length of the line segment obtained;
inserting the injection part into the minute object along the insertion direction; and
injecting the material from the injection part into the minute object.

22. The manufacturing method according to claim 21, wherein the obtaining step includes the steps of:
measuring a shape of the minute object; and
determining the length of the line segment of the minute object based on the shape of the minute object measured.

23. The manufacturing method according to claim 21 or 22, wherein the determining step includes the steps of:
selecting the longest line segment from among plural line segments that have been obtained; and
determining, as the insertion direction, a direction that provides the longest line segment selected.

24. A manufacturing method of a minute object into which a material has been injected, the manufacturing method comprising the steps of:
obtaining an angle between a surface of the minute object and a line segment in the minute object which passes a target position in the minute object;
determining an insertion direction for an injection part that injects the material into the minute object, based on the angle obtained;
inserting the injection part into the minute object along the insertion direction; and -
injecting the material from the injection part into the minute object.

25. The manufacturing method according to claim 24, wherein the obtaining step includes the steps of:
obtaining an angle between the surface of the minute object and each of plural line segments in the minute object which pass a target position in the minute object; and
determining one of the plural line segments that is closest to a normal to the surface of the minute object,
wherein the step of determining the insertion direction determines, as the insertion direction, a direction of the one of the plural line segments.

26. A manufacturing method of a minute object into which a material has been injected, the manufacturing method comprising the steps of:
obtaining a shape of a first part in the minute object, and a shape of a second part in the first part;
determining one of plural line segments in the minute objects which one does not pass the second part;
inserting an injection part that injects the material into the minute object, into the object along a direction of the one of plural line segments; and
injecting the material from the injection part into the minute object.

27. A manufacturing method of a minute object into which a material has been injected, the manufacturing method comprising the steps of:
obtaining a shape of a first part in the minute objects and a shape of a second part in the first part;
determining one of plural line segments in the minute objects which one passes the second part;
inserting an injection part that injects the material into the second part, into the object along a direction of the one of plural line segments; and
injecting the material from the injection part into the second part.

28. A manufacturing method of a minute object into which a material has been injected, the manufacturing method comprising the steps of:
obtaining a projected area of the minute object;
determining an insertion direction for an injection part that injects the material into the minute object, based on the projected area obtained;
inserting the injection part into the minute object along the insertion direction; and
injecting the material from the injection part into the minute object.

29. The manufacturing method according claim 28, wherein the obtaining step obtains projected areas in plural directions, and the determining step determines, as the insertion direction, a direction of the smallest projected area.

30. A manufacturing method of a minute object into which a material has been injected, the manufacturing method comprising the steps of:
obtaining a shape of the minute object;
obtaining shapes of inscribed solids in the minute object, based on the shape of the minute object obtained;
determining the largest inscribed solid;
inserting an injection part into the minute object toward the largest inscribed solid; and
injecting the material from the injection part into the minute object.

31. The manufacturing method according to claim 31, further comprising the step of determining a center of the largest inscribed solid, wherein the inserting step inserts the injection part toward the center determined.

32. The manufacturing method according claim 30 or 31, wherein the solid is a sphere.
